# EUROPEAN PATENT SPECIFICATION

(11) **EP 4 178 526 B1**
(45) Date of publication and mention of the grant of the patent: **18.11.2026**
(21) Application number: 21736331.6
(22) Date of filing: 01.07.2021
(51) Int. Cl.: A61K 8/04, A61K 8/73, A61K 8/92

(54) **OIL-CONTAINING WASHING COMPOSITION**
ÖLENTHALTENDE WASCHZUSAMMENSETZUNG
COMPOSITION LAVANTE CONTENANT DE L'HUILE

(30) Priority: 07.07.2020 EP 20184511
(43) Date of publication of application: 17.05.2023
(73) Proprietor: Bayer Consumer Care AG, 4052 Basel (CH)
(72) Inventor: ROUVIERE, Juliette, 74240 Gaillard (FR); TAYLOR, Richard, 74240 Gaillard (FR); BELLAMY, Adèle, 74240 Gaillard (FR); DIRLEWANGER, Laure, 74240 Gaillard (FR)
(74) Representative: BIP Patents
(86) International application number: PCT/EP2021/068144
(87) International publication number: WO 2022/008334

(56) References cited:
- WO-A1-03/084501
- JP-A- 2019 202 963
- US-B2- 9 217 127
- GAO Q ET AL: "Characterisation of acidic heteroglycans from Tremella fuciformis Berk with cytokine stimulating activity", CARBOHYDRATE RESEARCH, PERGAMON, GB, vol. 288, 19 July 1996 (1996-07-19), pages 135 - 142, XP004018883, ISSN: 0008-6215, DOI: 10.1016/0008-6215(96)00091-2

## Description

The invention relates to a transparent and moisturizing cosmetic wash compositions containing oil.

It is highly desirable for consumers to have cosmetic washes or cleansing compositions which comprise natural oils since those oils can provide many different cosmetic benefits. The achieved effect directly depends on the respective natural-oil used.

It is highly desirable for consumers to have cosmetic washes or cleansing compositions which do not damage their skin. Typically, cosmetic wash preparations are aqueous formulations containing surfactants that can be delipidating or even irritating. Moreover, washes induce pH change to the skin that can cause severe skin damage. Consumers are looking for a solution that would provide cleansing and foaming without drying out or damaging their skin. The second challenge is that cosmetic wash compositions are often aqueous formulations which makes the introduction of oils into these aqueous formulations especially challenging because natural oils providing cosmetic effects are immiscible with water resulting in bi-phasic products. One possible way to address that challenge is to formulate oil-in-water emulsions. Another way is to formulate a bi-phasic product which has to be shaken before application wherein the shaking creates an unstable and turbid emulsion which separates over time again. Both options are well-known in the technical field of cosmetic wash and cleansing compositions.

Both options have significant drawbacks e.g. O/W and W/O emulsions are usually not transparent often resulting in opaque primary packaging so that the consumer cannot see the actual formulation at the store at which she or he has to take a decision on which wash or cleansing product to buy. Bi-phasic formulations as described above always have to be shaken before application which is an additional step to carry out and may be seen as inconvenient. In addition, depending on the shaking behavior of each consumer the mixture may not be homogenous. JP2019202963 describes a viscous cosmetic gel composition, which may contain gums.

Thus, for consumers, homogeneous cosmetic wash or cleansing products which contain a cosmetically-beneficial plant-derived oil, are transparent and easily applicable with no additional preparation steps before application are highly desirable. In addition, a cosmetic wash that at the same time has moisturizing properties is even more desirable.

Researchers have surprisingly found that a cosmetic wash composition in form of a gel comprising:
a. xanthan gums in an amount of 0.05 to 1 wt.-% of the total composition, and
b. guar gums 0.1 to 2.5 % by weight of the total composition, and
c. a pre-solubilized plant-derived oil, and
d. 1 to 8 surfactant(s), and
e. water
wherein the composition is free of inorganic and/or organic sulfates and free of polyethylene glycol and/or polyethylene glycol-derivatives and wherein the cosmetic wash composition has a viscosity of 0.5 to 5 Pa s, more preferably of 0.8 to 2.5 Pa s fulfills all the above objects.

All percentages, ratios and proportions herein are by weight, unless otherwise specified. All temperatures are in degrees Celsius (°C) unless otherwise specified. The expression "room temperature" may be used synonymously for 20 °C. If any unit or condition is not explicitly defined in the context of the present application a person skilled in the art will always consider the most obvious unit or condition. If in the context of the application any unit or condition is missing, and this unit or condition is defined in another section of the application the same unit or condition applies where it is missing.

The cosmetic wash composition according to the invention comprises an anionic polysaccharide selected from xanthan gums. The anionic polysaccharide according to the invention which is a xanthan gum generally has a molecular weight of from 0.4 million to 25 million g mol⁻¹. Its viscosity generally ranges from 0.85 to 1,700 Pa s when measured at 25 °C using a 1% solution of the gum in 1% KCI, on a viscometer of the Brookfield LV type, at 60 rpm using Spindle No. 3.

Xanthan gum may be generally characterized as an anionic heteropolysaccharide, with a primary structure consisting of repeating penta-saccharide units formed by two glucose units, two mannose units, and a glucuronic acid unit. These repeating penta-saccharide units give xanthan gum its characteristic backbone, which consists of (1→4) β-D-glucopyranosyl units substituted at C-3 on every other glucose residue with a charged trisaccharide sidechain. The trisaccharide sidechain consists of a D-glucuronic acid unit between two D-mannose units. Slightly less than half (about 40%) of the terminal D-mannose residues contain a pyruvic acid residue linked via keto groups to the four and six positions, and the D-mannose linked to the main chain mostly contains an acetyl group at position O-6. Some side chains may be missing. The acetate and pyruvate contents are variable on the side chain, and depend on the bacterial strains and on the fermentation conditions used to produce the gum.

In a preferred embodiment the xanthan gum according to the invention has an average particle size of 50 to 1250 µm.

Xanthan gum is available from several commercial suppliers such as RT Vanderbilt Company, Jungbunzlauer International AG, Unionchem, KahlWax and CP Kelco. Examples of suitable xanthan gums are e.g. Keltrol^{®}, Keltrol^{®} CG-T, Keltrol^{®} F, Keitrol^{®} T, Keltrol^{®} TF, Xantural^{®} 180 and Vanzan^{®} NF. For a person skilled in the art it is obvious that the above lists of suppliers and xanthan gums are non-exhaustive and not limiting the invention in any way.

The anionic polysaccharide which is selected from xanthan gum is comprised in an amount of 0.001 to 5 % by weight of the total composition, more preferably 0.01 to 2.5 wt.-% and even more preferably 0.05 to 1 wt.-% of the total composition.

The cosmetic wash composition according to the invention also comprises a cationic polysaccharide selected from guar gums.

In general, for the purposes of the present invention, the term guar gum is defined as any guar gum containing cationic groups and/or groups which are ionizable into cationic groups in the cosmetic wash composition according to the invention and the pH range useful for a cosmetic wash composition. A pH range particularly relevant is from 4 to 10 and more preferably 4.5 to 6. The cationic groups are chosen from those comprising primary amine groups, secondary, tertiary and/or quaternary.

Cationic guar gums used generally have a weight average molecular weight between 5x10⁴ and 8x10⁶, and preferably between 10³ and 3x10⁶ g mol⁻¹.

According to the Invention, it is preferable to use a guar gum modified by hydroxypropyl or hydroxypropyl trimethylammonium groups. Such products are also sold under the trade names JAGUAR Excel, JAGUAR HP105, JAGUAR C13 sec, JAGUAR the C 15, JAGUAR 17 and JAGUAR C162 by the companies, Solvay, BASF and Cosphatech LLC. For a person skilled in the art it is obvious that the above lists are non-exhaustive and do not limit the invention in any way. The most preferred guar gums are guar hydroxypropyl trimethylammonium chloride with the CAS number 65497-29-2 or hydroxypropyl guar with the CAS number 39421-75-5.

Cosmetic wash compositions according to the invention comprise the cationic polysaccharide in an amount of 0.001 to 10 % by weight of the total composition, more preferably 0.01 to 5 % by weight of the total composition, even more preferably 0.1 to 2.5 % by weight of the total composition.

The combination of the anionic and the cationic polysaccharides results in the formation of a specifically desired texture of the cosmetic wash composition which can be best described as a fluid gel. In addition, only the combination of the anionic and the cationic polysaccharides together with the pre-solubilized plant-derived oil resulted in the formation of a transparent gel formulation, wherein usually the introduction of oils results in turbid formulations.

Without being bound by theory the inventors believe that the combination and the amounts of the polysaccharides as defined above are mainly responsible for the moisturizing properties of the cosmetic washes of the invention. The inventors also observed that the plant-derived oil alone was not the key ingredient to explain the overall very good moisturizing properties of the cosmetic washes of the present invention, but rather the combination of all features leads to the moisturizing properties. The surfactant blend was also observed to have a supporting effect on the moisturizing properties of the cosmetic wash compositions of the invention.

As used herein a gel means a topical dosage form for dermatological application that is a semisolid dosage form that contains at least one gelling agent to provide stiffness to a solution or colloidal dispersion (Buhse et al., Int. J. Pharm. 2005, 295, 101-112). A gel may also comprise suspended particles. Gels according to this invention are pourable and flow at low shear stress and conform to their container at 20 °C. A conventional gel also contains more than 50 % by weight of water and other volatiles, wherein the content of water and volatiles can be measured by loss on drying test by heating at 105 °C until constant weight is achieved. Cosmetic wash compositions according to the invention preferably comprise water in an amount of at least 40 % by weight of the total composition, more preferably at least 50 % by weight of the total composition and even more preferable at least 60 % of the total composition.

In general, a gel has a specific viscosity range which is provided for the cosmetic wash composition by the anionic and the cationic polysaccharides. Thus, the cosmetic wash composition according to the invention has a viscosity from 0.5 to 5 Pa s. In a preferred embodiment the viscosity is from 0.8 to 2.5 Pa s.

A plant-derived oil according to the invention is an oil that is obtainable by extraction from plants. The plant-derived oil may be a complex mixture of multiple oil components or it may be a single oil component. Especially if the plant-derived oil is a single oil component or a mixture of a limited number of components the oil component(s) may be manufactured synthetically.

A plant-derived oil for use in the cosmetic wash compositions according to the invention are preferably selected from argan oil, castor oil, inca inchi oil, sweet almond oil, apricot oil, sunflower oil, olive oil, soy bean oil, safflower oil, cotton seed oil, corn oil , walnut oil, peanut oil, avocado oil, jojoba, palm oil, sesame oil, anise oil, lemon oil, orange oil, rosemary oil, wintergreen oil, thyme oil, lavender oil, cloves oil, hops oil, tea tree oil, citronella oil, wheat oil, barley oil, lemongrass oil, cedar leaf oil, cedarwood oil, cinnamon oil, fleagrass oil, geranium oil, sandalwood oil, violet oil, cranberry oil, eucalyptus oil, vervain oil, peppermint oil, basil fennel oil.

Plant-derived oils are highly effective substances that possess an array of different effects. The fragrance emanated from oils may affect the limbic system in the brain, e.g. by processing feeling. Therefore, some oils have a mood-enhancing effect. Individual molecules are transported into the bloodstream through the skin where they are integrated into the cell membrane, influencing bodily processes. As a result, these molecules can promote the renewal of cells or stimulate the metabolic processes. Plant-derived oils are primarily used in natural cosmetics due to their emollient, antiinflammatory and anti-oxidant (vitamin E content) properties, and in addition they may have antibacterial and/or antiviral properties. They may also serve as natural preserving agent.

The term "pre-solubilized" in the context of the inventions and in particular in the context together with the expression "plant-derived oil" means that the plant-derived oil is solubilized in an aqueous phase so that the plant-derived oil and an aqueous phase build a mono phasic system. Plant-derived oils can for example be pre-solubilized by the process of saponification, neutralization, salification, esterification and ionization of the plant-derived oil component(s) as it is done by the AQUASILOIL^{®} technology marketed by Greentech GmbH. As an alternative also Floraesters^{®} from Floratech, which also utilize chemically modified plant-derived oils may be regarded as pre-solubilized plant-derived oils. An advantage of these technologies is that the plant-derived oil itself acts as a surfactant-like compound, thus the amount of solubilizers or surfactants or the number and kind of different solubilizers and surfactants can be reduced which helps to decrease irritation risks and to decrease the environmental impact factor.

The preferred amount of pre-solubilized plant-derived oil in the cosmetic wash composition according to this invention is from 0.001 to 10 % of active by weight of the total composition, more preferred from 0.01 to 5 % of active matter by weight, even more preferred from 0.01 to 2.5 % of active matter by weight and most preferred from 0.01 to 1 % by weight of the total composition of active matter.

As mentioned above the combination of the mild surfactants, anionic and the cationic polysaccharide together with the pre-solubilized plant-derived oil results in a transparent and moisturizing aqueous gel containing a cosmetically beneficial oil.

In other cosmetic wash compositions, which are known in the prior art oils also have been used and some of them may also have been transparent. But these cosmetic wash compositions comprised additional solubilizer and especially polyethylene glycol and/or polyethylene glycol-derivatives. Polyethylene glycol and/or polyethylene glycol-derivatives like e.g. propylene glycol are deemed to have certain disadvantages especially for already damaged skin. Studies show that Polyethylene glycol and/or polyethylene glycol-derivatives have an enhanced penetration effect by altering the surface tension of the skin. Thus, PEGs may upset the natural moisture balance. In addition, PEGs are not always pure, but often come contaminated with a host of toxic impurities. Both effects taken together - the enhanced skin penetration of PEG containing composition and the impurities - may cause skin irritation or skin sensitization. As a consequence, the cosmetic wash compositions of the present invention are free of polyethylene glycol and/or polyethylene glycol-derivatives.

Since the compositions of the present invention are cosmetic wash compositions, they also comprise at least one surfactant. A wide variety of surfactants may be used, but preferred surfactants are chosen from anionic surfactants, amphiphilic surfactants and nonionic surfactants. It is even more preferred to use at least one anionic surfactant and one nonionic surfactant. In the most preferred embodiment of the invention the cosmetic wash composition comprises at least one glucoside surfactant.

Preferred anionic surfactants may be selected from disodium cocoyl glutamate, sodium lauryl sarcosinate, sodium stearte, acyl glycinates, sodium stearate or sodium gluconate.

Preferred amphiphilic surfactants may be selected from capryl/capramidopropyl betaine, sodium cocoamphoacetate or cocoamidopropylbetaine.

Preferred nonionic surfactants may be selected from caprylyl/capryl glucoside, lauryl glucoside or alkyl polyglucoside.

In a preferred embodiment the cosmetic wash composition according to the invention comprises surfactant(s) in an amount from 0.1 to 20 % by weight of the total composition, wherein the amount is based on the active material of the commercially available raw materials.

Cosmetic wash compositions according to the invention may contain at least one vitamin or vitamin derivative in an amount of 0.001 to 15 % by weight of the total composition, more preferably from 0.01 to 10 % by weight of the total composition and even more preferably from 0.1 to 7.5 % by weight of the total composition. Vitamins that are typically used in cosmetics are for example vitamin C (ascorbic acid and its derivatives), vitamin E (tocopherol and its derivatives), vitamin B3 (Niacinamide and derivatives), vitamin B5 (Panthenol and derivatives) or vitamin A (retinal, retinol, retinoic acid and retinyl palmitate and its derivatives).

Cosmetic wash compositions according to the invention comprise water in an amount of at least 40 % by weight of the total composition, more preferably at least 50 % by weight of the total composition and even more preferable at least 60 % of the total composition.

Cosmetic wash compositions according to the invention may be used to moisturize the skin. Moisturization is defined as an increase of the arbitrary units of corneometric measurements as carried out in the experimental section.

The cosmetic wash compositions of the present invention are of course not limited to the active ingredients as described above, but can comprise fillers, dyes, solvents, stabilizers, fragrancing agents, lubricants, humectants, thickeners or any other auxiliary useful for the present invention.

### Examples

### Formulas fit within the scope:

| **Ingredients (according to INCI)** | **1** | **2** | **3** |
|---|---|---|---|
| Water | QSP | | |
| CAPRYL/CAPRAMIDOPROPYL BETAINE | 0 | 3% to 7% | 0 |
| COCAMIDOPROPYL BETAINE | 0 | 0 | 3% to 7% |
| CAPRYL/CAPRYL GLUCOSIDE | 1% to 3% | 0 | 0 |
| DISODIUM COCOYL GLUTAMATE | 2% to 4% | 2% to 4% | 0 |
| LAURYL GLUCOSIDE | 2% to 5% | 2% to 5% | 2% to 5% |
| SODIUM COCOAMPHOACETATE | 2% to 4% | 0 | 2% to 4% |
| SODIUM GLUCONATE | 0 | 0 | 2% to 4% |
| DEXPANTENOL | 0% to 3% | | |
| GLYCERINE | 1% to 3% | | |
| 1,2-HEXANDIOL | 1% to 2% | | |
| GUAR HYDROXYPROPYLTRIMONIUM CHLORIDE | 0.1% to 1% | | |
| CITRIC ACID | 0.1% to 6% | | |
| NIACINAMIDE | 0% to 1% | | |
| LYSINE* | 0.01% to 0.05% | | |
| ARGIANIA SPINOSA KERNEL OIL* | 0.01% to 0.05% | | |
| SODIUM CHLORIDE* | 0.001% to 0.01% | | |
| XANTHAN GUM | 0.1% to 0.5% | | |
| | | | |
| Viscosity (RS6000 cone/plan C60/1° Ti L shear rate 10 s⁻¹) | 0.8 to 2.5 Pa s | 0.8 to 2.5 Pa s | 0.8 to 2.5 Pa s |
| Appearance | Clear and homogeneous | Clear and homogeneous | Clear and homogeneous |
| Irritating potential | No | No | No |

| | | | |
|---|---|---|---|
| *These ingredients have been added in the form of a pre-solubilized oil commercially available under the brand name Aquasil Oil^{®} | | | |

### Formulas don't fit in the scope:

| **Ingredients (according to INCI)** | **4** | **5** | **6** |
|---|---|---|---|
| Water | QSP | | |
| CAPRYL/CAPRAMIDEOPROPYL BETAINE | 3% to 7% | 3% to 7% | 3% to 7% |
| CAPRYL/CAPRYL GLUCOSIDE | 0 | 0 | 0 |
| DISODIUM COCOYL GLUTAMATE | 2% to 4% | 0 | 0 |
| LAURYL GLUCOSIDE | 2% to 5% | 0 | 2% to 5% |
| SODIUM COCOAMPHOACETATE | 0 | 0 | 2% to 5% |
| SODIUM LAURYL SULFATE | 0 | 3% to 7% | 0 |
| DEXPANTENOL | 1% to 3% | | |
| GLYCERINE | 1% to 3% | | |
| 1,2-HEXANDIOL | 1% to 2% | | |
| GUAR HYDROXYPROPYLTRIMONIUM CHLORIDE | 0 | 0.1% to 1% | 0 |
| CITRIC ACID | 0% to 6% | | |
| NIACINAMIDE | 0% to 2% | | |
| LYSINE* | 0 | 0.01% to 0.05% | 0.01% to 0.05% |
| ARGIANIA SPINOSA KERNEL OIL* | 2% to 5% | 0.001% to 0.01% | 0.1% to 0.5% |
| SODIUM CHLORIDE* | 0 | 0.001% to 0.01% | 0.001% to 0.01% |
| HYDROXYETHYLACRYLATE | 0 | 0 | 0.1% to 0.5% |
| SODIUM ACRYLOYL DIMETHYL TAURATE COPOLYMER | 0 | 0 | 0.1% to 0.5% |
| XANTHAN GUM | 1% | 0.1% to 0.5% | 0.1% to 0.5% |
| | | | |
| Viscosity (RS6000 cone/plan C60/1° Ti L shear rate 10 s⁻¹) | 0.8 to 2.5 Pa s | 0.8 to 2.5 Pa s | < 0 Pa s |
| Appearance | turbid | clear | turbid |
| Irritating potential | no | yes | no |

| | | | |
|---|---|---|---|
| *These ingredients have been added in the form of a pre-solubilized oil commercially available under the brand name Aquasil Oil^{®} | | | |

### Corneometry

The hydration level of the upper layers of the skin was tested via corneometry, a technique well established in the art and known by a person skilled in the art. Every commercially and calibrated corneometer may be used for testing the hydration level since hydration level always is conducted against an untreated control measurement, which may be either the measurement right before applying a test composition for the first time to a skin area or parallel measurements of a treated and an untreated area of the skin over the complete test period.

The hydration level of the upper layers of the skin is expressed in arbitrary units (Corneometric indices) ranging from approximately 0 (no hydration) to 120 (very high hydration).

For each area and for each experimental time (Ti), the following individual data is calculated:
- means of the triplicate Corneometric indices,
- absolute variations (in comparison with T0): Δ = Ti-T0,
- relative variations (in comparison with T0): Δ% = [(Ti-T0) x 100] / T0,
- Differences between treated area and control for each time point.

For the absolute variations in comparison with T0 (Δ = Ti-T0), the following parameters are defined or calculated for treated area and negative control and for each experimental time Ti: number of subjects (n), mean, standard deviation, median, minimum, maximum.

A global comparison over time (ANOVA / Friedman test) of the instrumental measurements data for each test area will be performed. The ANOVA / Friedman test results in a p-value which is an indicator of significance. If the p-value of the ANOVA / Friedman test is p <= 0.05 the result/effect is considered as being statistically relevant.

A total of 42 subjects (male or female) were included in the study and all subjects completed the study which means that all subjects applied the composition of the present invention twice a day for at least 14 days on the face for compositions according to example 1 and/or on a defined area on a forearm for composition according to example 2 above.

The 42 subjects included in the study had a mean age of 42 years (range from 42 to 70 years) and there were 95.2% women and 4.8% men and phototype II, III and IV represented 16.7%, 73.8% and 9.5%, respectively.
a) In this test, a comparison between a skin area that was treated with a composition according to example 2 with an untreated skin area was carried out. Given are the mean values of all 42 tested subjects and the mean difference between the treated and the untreated skin area. If a result is significant was calculated via the ANOVA / Friedman test and a p-value of < 0.05 was considered to be significant.

| | **mean value treated area** | **mean value untreated area** | **mean difference** | **p-value** (significant at p < 0.05) | |
|---|---|---|---|---|---|
| **D0** | 24.83 | 25.61 | 0.78 | 0.537 | |
| **D0/T1H** | 27.44 | 25.43 | 2.01 | 0.083 | |
| **D0/T2H** | 28.11 | 25.70 | 2.40 | 0.059 | |
| **D0/T5H** | 29.66 | 26.65 | 3.01 | 0.015 | Significant difference |
| **D1** | 27.79 | 25.44 | 2.35 | 0.074 | |
| **D7** | 27.81 | 24.95 | 2.86 | 0.030 | Significant difference |
| **D14** | 28.07 | 25.70 | 2.37 | 0.061 | |

b) In this test, the control was the corneometric measurement carried out right before applying the cosmetic wash of example 1 for the first time. Given is the mean value of the 42 subjects before the first application of the product, the mean difference and the p-value according to the ANOVA / Friedman test, wherein for this measurements results having a p-value of < 0.0125 were considered as statistically significant.

| **treated area vs. D0** | **Mean values** | **mean difference** | **p-value** (significant at p < 0.0125) | |
|---|---|---|---|---|
| **D0** | 33.31 | - - | - - | - - |
| **D0/T1H-D0** | -- | 14.05 | <0.001 | Significant increase |
| **D0/T2H-D0** | -- | 15.85 | <0.001 | Significant increase |
| **D0/T5H-D0** | -- | 18.13 | <0.001 | Significant increase |
| **D1-D0** | -- | 13.70 | <0.001 | Significant increase |
| **D7-D0** | -- | 16.25 | <0.001 | Significant increase |
| **D14-D0** | -- | 16.55 | <0.001 | Significant increase |

Typically, cosmetic wash compositions in general tend to stress the skin e.g. because of the surfactants and other ingredients used to clean the skin which often leads to dry skin. Surprisingly, the cosmetic washes of the present invention replenish and maintain the skin's natural moisture level as can be seen in the corneometric measurements above.

## Claims

1. Cosmetic wash composition in form of a transparent gel comprising:
a. an anionic polysaccharide, comprised in an amount of 0.05 to 1 wt.-% of the total composition and
b. a cationic polysaccharide, comprised in an amount of 0.1 to 2.5 % by weight of the total composition and
c. a pre-solubilized plant-derived oil, and
d. 1 to 8 surfactant(s), and
e. water in an amount of at least 60 % by weight of the total composition
wherein the anionic polysaccharide is selected from xanthan gums, the cationic polysaccharide is selected from guar gums, the composition is free of inorganic and/or organic sulfates and free of polyethylene glycol and/or polyethylene glycol-derivatives and wherein the cosmetic wash composition has a viscosity of 0.5 to 5 Pa s, more preferably of 0.8 to 2.5 Pas and wherein viscosity is determined using RS6000 cone/plan C60/1 ° Ti L shear rate 10 s⁻¹.

2. Cosmetic wash composition according to claim 1 **characterized in that** the anionic polysaccharide is a xanthan gum having an average particle size of 50 to 1250 µm.

3. Cosmetic wash composition according to any of claims 1 to 2 **characterized in that**, preferably the cationic polysaccharide is guar hydroxypropyltrimonium chloride and most preferred the cationic polysaccharide is guar hydroxypropyltrimonium chloride with the CAS number 65497-29-2 or hydroxypropyl guar with the CAS number 39421-75-5.

4. Cosmetic wash composition according to any of claims 1 to 3 **characterized in that** the plant derived oil is selected from argan oil, castor oil, inca inchi oil, sweet almond oil, apricot oil, sunflower oil, olive oil, soy bean oil, safflower oil, cotton seed oil, corn oil , walnut oil, peanut oil, avocado oil, jojoba, palm oil, sesame oil, anise oil, lemon oil, orange oil, rosemary oil, wintergreen oil, thyme oil, lavender oil, cloves oil, hops oil, tea tree oil, citronella oil, wheat oil, barley oil, lemongrass oil, cedar leaf oil, cedarwood oil, cinnamon oil, fleagrass oil, geranium oil, sandalwood oil, violet oil, cranberry oil, eucalyptus oil, vervain oil, peppermint oil, basil fennel oil.

5. Cosmetic wash composition according to any of claims 1 to 4 **characterized in that** it comprises at least one vitamin in an amount of0.001 to 15 % by weight of the total composition, more preferably 0.01 to 10 % by weight of the total composition and even more preferably of0.1 to 7.5 % by weight of the total composition.

6. Cosmetic wash composition according to any of the claims 1 to 5 **characterized in that** it comprises at least one surfactant chosen from anionic surfactants, amphiphilic surfactants and nonionic surfactants.

7. Cosmetic wash composition according to any of the claims 1 to 6 **characterized in that** it comprises at least one anionic surfactant and one nonionic surfactant.

8. Cosmetic wash composition according to any of the claims 1 to 7 **characterized in that** it comprises at least one glucoside surfactant.

9. Cosmetic wash composition according to any of the claims 1 to 8 **characterized in that** it comprises surfactant(s) in an amount from 0.1 to 20 % by weight of the total composition, wherein the amount is based on the active material.

10. Non-therapeutic use of the cosmetic wash composition according to any of claims 1 to 9 for moisturizing the skin.

## Patentansprüche

1. Kosmetische Waschzusammensetzung in Form eines transparenten Gels, umfassend:
a. ein anionisches Polysaccharid, enthalten in einer Menge von 0,05 bis 1 Gew.-% der Gesamtzusammensetzung, und
b. ein kationisches Polysaccharid, enthalten in einer Menge von 0,1 bis 2,5 Gew.-% der Gesamtzusammensetzung, und
c. ein vorgelöstes pflanzliches Öl, und
d. 1 bis 8 Tensid(e), und
e. Wasser in einer Menge von mindestens 60 Gew.-% der Gesamtzusammensetzung,
wobei das anionische Polysaccharid aus Xanthangummis ausgewählt ist, das kationische Polysaccharid aus Guargummis ausgewählt ist, die Zusammensetzung frei von anorganischen und/oder organischen Sulfaten und frei von Polyethylenglykol und/oder Polyethylenglykol-Derivaten ist, und wobei die kosmetische Waschzusammensetzung eine Viskosität von 0,5 bis 5 Pa s, bevorzugter von 0,8 bis 2,5 Pa s aufweist, und wobei die Viskosität unter Verwendung von RS6000 Kegel/Platte C60/1° Ti L Scherrate 10 s⁻¹ bestimmt wird.

2. Kosmetische Waschzusammensetzung nach Anspruch 1, **dadurch gekennzeichnet, dass** das anionische Polysaccharid ein Xanthangummi mit einer mittleren Teilchengröße von 50 bis 1250 µm ist.

3. Kosmetische Waschzusammensetzung nach einem der Ansprüche 1 bis 2, **dadurch gekennzeichnet, dass** vorzugsweise das kationische Polysaccharid Guar-Hydroxypropyltrimoniumchlorid ist und höchst bevorzugt das kationische Polysaccharid Guar-Hydroxypropyltrimoniumchlorid mit der CAS-Nummer 65497-29-2 oder Hydroxypropylguar mit der CAS-Nummer 39421-75-5 ist.

4. Kosmetische Waschzusammensetzung nach einem der Ansprüche 1 bis 3, **dadurch gekennzeichnet, dass** das pflanzliche Öl ausgewählt ist aus Arganöl, Rizinusöl, Inca-Inchi-Öl, Süßmandelöl, Aprikosenkernöl, Sonnenblumenöl, Olivenöl, Sojaöl, Distelöl, Baumwollsamenöl, Maisöl, Walnussöl, Erdnussöl, Avocadoöl, Jojobaöl, Palmöl, Sesamöl, Anisöl, Zitronenöl, Orangenöl, Rosmarinöl, Wintergrünöl, Thymianöl, Lavendelöl, Nelkenöl, Hopfenöl, Teebaumöl, Citronellaöl, Weizenöl, Gerstenöl, Zitronengrasöl, Zedernblattöl, Zedernholzöl, Zimtöl, Poleiöl, Geranienöl, Sandelholzöl, Veilchenöl, Cranberryöl, Eukalyptusöl, Eisenkrautöl, Pfefferminzöl, Basilikum-Fenchelöl.

5. Kosmetische Waschzusammensetzung nach einem der Ansprüche 1 bis 4, **dadurch gekennzeichnet, dass** sie mindestens ein Vitamin in einer Menge von 0,001 bis 15 Gew.-% der Gesamtzusammensetzung, bevorzugter 0,01 bis 10 Gew.-% der Gesamtzusammensetzung und noch bevorzugter 0,1 bis 7,5 Gew.-% der Gesamtzusammensetzung umfasst.

6. Kosmetische Waschzusammensetzung nach einem der Ansprüche 1 bis 5, **dadurch gekennzeichnet, dass** sie mindestens ein Tensid umfasst, ausgewählt aus anionischen Tensiden, amphiphilen Tensiden und nichtionischen Tensiden.

7. Kosmetische Waschzusammensetzung nach einem der Ansprüche 1 bis 6, **dadurch gekennzeichnet, dass** sie mindestens ein anionisches Tensid und ein nichtionisches Tensid umfasst.

8. Kosmetische Waschzusammensetzung nach einem der Ansprüche 1 bis 7, **dadurch gekennzeichnet, dass** sie mindestens ein Glucosid-Tensid umfasst.

9. Kosmetische Waschzusammensetzung nach einem der Ansprüche 1 bis 8, **dadurch gekennzeichnet, dass** sie Tensid(e) in einer Menge von 0,1 bis 20 Gew.-% der Gesamtzusammensetzung umfasst, wobei die Menge auf dem Wirkstoffgehalt basiert.

10. Nicht-therapeutische Verwendung der kosmetischen Waschzusammensetzung nach einem der Ansprüche 1 bis 9 zur Befeuchtung der Haut.

## Revendications

1. Composition cosmétique lavante sous forme d'un gel transparent comprenant :
a. un polysaccharide anionique, compris en une quantité de 0,05 à 1 % en poids de la composition totale, et
b. un polysaccharide cationique, compris en une quantité de 0,1 à 2,5 % en poids de la composition totale, et
c. une huile d'origine végétale pré-solubilisée, et
d. 1 à 8 tensioactif(s), et
e. de l'eau en une quantité d'au moins 60 % en poids de la composition totale,
dans laquelle le polysaccharide anionique est choisi parmi les gommes xanthane, le polysaccharide cationique est choisi parmi les gommes guar, la composition est exempte de sulfates inorganiques et/ou organiques et exempte de polyéthylène glycol et/ou de dérivés de polyéthylène glycol, et dans laquelle la composition cosmétique lavante présente une viscosité de 0,5 à 5 Pa s, de préférence de 0,8 à 2,5 Pa s, et dans laquelle la viscosité est déterminée à l'aide du RS6000 cône/plateau C60/1° Ti L taux de cisaillement 10 s⁻¹.

2. Composition cosmétique lavante selon la revendication 1, **caractérisée en ce que** le polysaccharide anionique est une gomme xanthane ayant une taille moyenne de particules de 50 à 1250 µm.

3. Composition cosmétique lavante selon l'une quelconque des revendications 1 à 2, **caractérisée en ce que**, de préférence, le polysaccharide cationique est le chlorure de guar hydroxypropyltrimonium et, de manière très préférée, le polysaccharide cationique est le chlorure de guar hydroxypropyltrimonium avec le numéro CAS 65497-29-2 ou l'hydroxypropyl guar avec le numéro CAS 39421-75-5.

4. Composition cosmétique lavante selon l'une quelconque des revendications 1 à 3, **caractérisée en ce que** l'huile d'origine végétale est choisie parmi l'huile d'argan, l'huile de ricin, l'huile d'inca inchi, l'huile d'amande douce, l'huile d'abricot, l'huile de tournesol, l'huile d'olive, l'huile de soja, l'huile de carthame, l'huile de graines de coton, l'huile de maïs, l'huile de noix, l'huile d'arachide, l'huile d'avocat, l'huile de jojoba, l'huile de palme, l'huile de sésame, l'huile d'anis, l'huile de citron, l'huile d'orange, l'huile de romarin, l'huile de gaulthérie, l'huile de thym, l'huile de lavande, l'huile de clou de girofle, l'huile de houblon, l'huile d'arbre à thé, l'huile de citronnelle, l'huile de blé, l'huile d'orge, l'huile de lemongrass, l'huile de feuille de cèdre, l'huile de bois de cèdre, l'huile de cannelle, l'huile de pouliot, l'huile de géranium, l'huile de santal, l'huile de violette, l'huile de canneberge, l'huile d'eucalyptus, l'huile de verveine, l'huile de menthe poivrée, l'huile de basilic-fenouil.

5. Composition cosmétique lavante selon l'une quelconque des revendications 1 à 4, **caractérisée en ce qu'**elle comprend au moins une vitamine en une quantité de 0,001 à 15 % en poids de la composition totale, de préférence de 0,01 à 10 % en poids de la composition totale et encore plus préférentiellement de 0,1 à 7,5 % en poids de la composition totale.

6. Composition cosmétique lavante selon l'une quelconque des revendications 1 à 5, **caractérisée en ce qu'**elle comprend au moins un tensioactif choisi parmi les tensioactifs anioniques, les tensioactifs amphiphiles et les tensioactifs non ioniques.

7. Composition cosmétique lavante selon l'une quelconque des revendications 1 à 6, **caractérisée en ce qu'**elle comprend au moins un tensioactif anionique et un tensioactif non ionique.

8. Composition cosmétique lavante selon l'une quelconque des revendications 1 à 7, **caractérisée en ce qu'**elle comprend au moins un tensioactif glucoside.

9. Composition cosmétique lavante selon l'une quelconque des revendications 1 à 8, **caractérisée en ce qu'**elle comprend un ou des tensioactif(s) en une quantité de 0,1 à 20 % en poids de la composition totale, la quantité étant basée sur la matière active.

10. Utilisation non thérapeutique de la composition cosmétique lavante selon l'une quelconque des revendications 1 à 9 pour hydrater la peau.
